# EUROPÄISCHE PATENTANMELDUNG

(11) **EP 0 845 264 A1**
(43) Veröffentlichungstag der Anmeldung: **03.06.1998**
(21) Anmeldenummer: 97118480.9
(22) Anmeldetag: 24.10.1997
(51) Int. Cl.: A61K 35/78

(54) **Teil- oder Vollextrakt aus nicht fermentierter Camellia sinensis L.**

(30) Priorität: 24.10.1996 CH 2606/96
(71) Anmelder: Emil Flachsmann AG, CH-8820 Wädenswil (CH)
(72) Erfinder: Kreuter, Matthias-Heinrich, Dr., 8880 Walenstadt (CH); Flachsmann, René, 8805 Richterswil (CH)
(74) Vertreter: Zink, Markus, Dr.

(57) **Zusammenfassung**

Die vorliegende Erfindung betrifft die Verwendung eines Teil- oder Vollextraktes aus nicht fermentierter Camellia sinensis L. zur Herstellung eines Medikamentes, eines Medizinproduktes, eines kosmetischen Präparates oder eines Nahrungsergänzungsproduktes, wobei diese Zubereitungen
- die Bildung von Nekrosen und/oder Atrophien in menschlichen oder tierischen Geweben und/oder das vorzeitige Absterben von vaskularisierten und nicht-vaskularisierten Zellen und Zellverbänden im menschlichen oder tierischen Körper verhindern oder zumindest wesentlich reduzieren, und
- sowohl die Adhäsion zwischen einzelnen, zum gleichen Gewebetyp gehörenden Zellen oder Zellverbänden fördern,
- als auch die Adhäsion zwischen einzelnen, zu verschiedenen nicht histo-kompatiblen Gewebetypen gehörenden Zellen oder Zellverbänden verhindern oder zumindest wesentlich reduzieren.

## Beschreibung

Die vorliegende Erfindung betrifft die Verwendung eines Teil- oder Vollextraktes aus nicht fermentierter Camellia sinensis L. zur Herstellung eines Medikamentes, eines Medizinproduktes, eines kosmetischen Präparates oder eines Nahrungsergänzungsproduktes.

Zubereitungen aus Camellia sinensis L. für medizinische und kosmetische Anwendung sind bekannt; siehe "Hagers Handbuch der Pharmazeutischen Praxis", Band 4, Drogen A-D, Springer-Verlag, (1992), Seiten 628 - 640.

Aufgrund seiner Inhaltsstoffe hat Camellia sinensis L. eine zentral anregende, leicht diuretische, je nach Extraktionszeit mehr oder weniger stark stopfende, die Herztätigkeit fördernde und möglicherweise antiarteriosklerotische Wirkung, siehe Stagg G.V., Millin D.J., (1975), J. Sci. Food. Agric. 26, Seiten 1439 - 1459.

Weiterer Stand der Technik betreffend Camellia sinensis L. ist beschrieben in Zeitschrift für Phytotherapie 17, (1995), Seiten 231 - 250.

So wird insbesondere den in Camellia sinensis L. enthaltenen Polyphenolen eine antioxidative Wirksamkeit zugeschrieben, die den menschlichen Körper vor sogenannten Radikalen schützen soll.

Ebenso wird den Polyphenolen eine entzündungshemmende Wirksamkeit zugeschrieben.

Eine Hemmung der Tumorentstehung mittels Camellia sinensis L. Extrakten ist in Tierversuchen belegt worden und wird durch epidemiologische Studien gestützt.

Daneben werden virostatische und bakteriostatische Wirkungen von Camellia sinensis L. Extrakten genannt.

In JP 07/101 837 wird ein Mittel mit Anti-Schuppen Aktivität beschrieben. Als aktiven Bestandteil enthält dieses Mittel einen alkoholischen Extrakt aus nicht naher spezifizierten Teeblättern.

In JP 06/056 687 wird ein Mittel beschrieben, mit welchem Zahnstein entfernt wird, und mit welchem die Bildung von Zahnstein verhindert wird.

Als aktive Bestandteile enthält dieses Mittel einen wässrigen oder einen mit einem hydrophilen organischen Lösungsmittel erhaltenden Extrakt von Teeblättern (Camellia sinensis).

Für die beanspruchte Aktivität wird die starke antimikrobielle Wirkung gegenüber periodontotischen pathogenen Bakterien verantwortlich gemacht.

In JP 08/073 350 wird ein Mittel für die Verbesserung von cerebralen Funktionen beschrieben.

Als aktiver Bestandteil wird Theanin (Glutaminsäureethylamid) genannt. Dieser Bestandteil kann in Tee-Extrakten enthalten sein.

In JP 06/100 442 wird ein Anti-Stress Mittel zur Verhinderung oder Linderung von mentalen und physikalen Krankheiten, hervorgerufen durch Stress, beschrieben.

Als aktiven Bestandteil enthält dieses Mittel L-Theanin natürlichen Ursprungs als solches oder in der Form eines pharmazeutisch annehmbaren Salzes, beispielsweise das Hydrochlorid.

In US PS 5 071 653 werden Verfahren zur Herstellung von Extrakten aus Camellia sinensis beschrieben.

Diese Extrakte fördern das Wachstum von Bifido-Bakterien.

Diese Extrakte enthalten im Gegensatz zu Teeaufgüssen, Teil- und Vollextrakten aus nicht fermentierter Camellia sinensis weder Flavonglykoside noch Polyphenole, ebenso keine Terpenoide und keine lipophilen Verbindungen.

Mit diesen in US PS 5 071 653 beschriebenen Verfahren werden hochselektive Faktionierungen von Camellia sinensis Inhaltsstoffen vorgenommen, die ein vollkommen unübliches Aktivitätsspektrum haben, nämlich die Wachstumsförderung von Darmbakterien.

Völlig überraschend wurden neue Verwendungsmöglichkeiten eines Teil- oder Vollextraktes aus nicht fermentierter Camellia sinensis L. gefunden.

Die vorliegende Erfindung betrifft die Verwendung eines Teil- oder Vollextraktes aus nicht fermentierter Camellia sinensis L. zur Herstellung eines Medikamentes, eines Medizinproduktes, eines kosmetischen Präparates oder eines Nahrungsergänzungsproduktes, wobei diese Zubereitungen
- die Bildung von Nekrosen und/oder Atrophien in menschlichen oder tierischen Geweben und/oder das vorzeitige Absterben von vaskularisierten und nicht-vaskularisierten Zellen und Zellverbänden im menschlichen oder tierischen Körper verhindern oder zumindest wesentlich reduzieren, und
- sowohl die Adhäsion zwischen einzelnen, zum gleichen Gewebetyp gehörenden Zellen oder Zellverbänden fördern,
- als auch die Adhäsion zwischen einzelnen, zu verschiedenen nicht histo-kompatiblen Gewebetypen gehörenden Zellen oder Zellverbänden verhindern oder zumindest wesentlich reduzieren.

Bevorzugte Ausführungsformen dieser Erfindung sind in den abhängigen Ansprüchen definiert.

Der für die weiter unten beschriebene Untersuchungen verwendete Extrakt von Camellia sinensis L. wurde wie folgt hergestellt.

6 kg getrocknete, nicht fermentierte Camellia sinensis L. (folia) wurden mit 60 kg eines Gemisches aus 8 Gewichtsteilen Ethanol und 2 Gewichtsteilen Wasser bei einer Temperatur von 25°C bis 35°C während 2 Stunden unter Rühren extrahiert.

Anschliessend wurde filtriert, und das Lösungsmittelgemisch wurde bei einem Druck von 50 mbar bis 150 mbar und einer Temperatur von 30°C bis 40°C bis zur Ethanolfreiheit eingeengt.

Aus dem so erhaltenen Konzentrat wurden gemäss EP 0 730 830 A1 unerwünschte lipophile Verunreinigungen und Rückstände entfernt.

Der so gereinigte Extrakt wurde anschliessend einem Verfahren zur Verminderung der Keimzahl unterworfen (30 Sekunden bei einer Temperatur von 120°C).

Anschliessend wurde dieser Extrakt sprühgetrocknet.

Man erhielt 1 kg nativen Trockenextrakt mit folgenden Analysendaten:
50 % m/m Phenylchromanderivate, berechnet als Epicatechin (HPLC),
6 % m/m Coffein (HPLC)
1 % m/m Theobromin (HPLC).

Ferner wurden qualitativ nachgewiesen:
Glutaminsäure-ethylamid
Flavonoide und
Pflanzensäuren (HPTLC).

Dieses Produkt ist bei der Firma Emil Flachsmann AG in CH-8820 Wädenswil/Schweiz unter der Bezeichnung "EFLA 85942" erhältlich.

Dieses Produkt wurde auf seine Wirkungen an einem besonderen Zellmodell, nämlich den multizellulären Sphäroiden, getestet.

Diese Sphäroide sind kugelförmige Zellaggregate, die in Suspensionskultur bei einem Durchmesser von etwa 1 mm bis zu 100'000 Zellen enthalten, und spiegeln die biologischen Verhältnisse von Zellverbänden in vivo in der Regel besser wider als herkömmliche Monolayer-Kulturen.

### Experiment 1

Getestet wurde der Einfluss des Produktes "EFLA 85942" auf das Volumenwachstum dieser Sphäroide als Funktion der Kultivierungsdauer und bei unterschiedlichen Konzentrationen von "EFLA 85942" im Vergleich zu Kontrollkulturen.

Es wurden folgende Ergebnisse erhalten.

Wie erwartet bewirkte "EFLA 85942" eine systematische und in weiten Bereichen der Wachstumsdauer eine signifikante Verringerung des Volumenwachstums der Sphäroide.

Völlig überraschend hingegen liess sich dabei nach Wirkungseintritt über einen weiten Konzentrationsbereich keine Konzentrationsabhängigkeit nachweisen.

Dieser Effekt deutet auf eine hohe therapeutische Breite hin.

Zudem wurde völlig überraschend festgestellt, dass die mit "EFLA 85942" behandelten Sphäroide während der gesamten Wachstumsphase keine nennenswerten Nekrosen bildeten.

In Kontrollkulturen, welche nicht mit "EFLA 85942" behandelt wurden, wurden bereits bei Durchmessern von etwa 200 Mikrometer zentrale Nekrosen festgestellt, die sich während der Wachstumsphase stark vergrösserten.

Am Ende der Versuchsdauer wiesen die unbehandelten Sphäroide nur noch eine sehr dünne vitale Randschicht auf.

Dieses Auftreten von Nekrosen ist typisch für das verwendete Versuchsmodell.

Dieses Verhalten der mit "EFLA 85942" behandelten Sphäroide wurde bis anhin noch nicht beobachtet.

Es wird vermutet, dass die bekannte antioxidative Wirkung der Polyphenole nicht alleine für dieses Verhalten verantwortlich sein kann.

Dafür sprechen auch Ergebnisse, welche aus begleitenden Untersuchungen an Einzelzellen erhalten wurden; siehe Experiment 2.

### Experiment 2

In einem ersten Versuch wurden Coloncarzinomzellen unter dem Einfluss von "EFLA 85942" in sogenannten adhärierenden Kulturflaschen ausgesät.

Erwartet wurde die Bildung einer sogenannten Monolayer-Schicht.

Völlig überraschend wurde aber die Bildung von dreidimensionalen, fest verbundenen Zellaggregaten beobachtet.

Auch dieses Verhalten wurde bis anhin noch nicht beobachtet.

Ausserdem wurden im Kulturmedium praktisch keine nicht-adhärierenden Einzelzellen beobachtet.

In einem zweiten Versuch wurden einige wenige Einzelzellen in nicht-adhärierenden Petrischalen ausgesät, mit dem Ziel, den Einfluss von "EFLA 85942" auf die Koloniebildungsfähigkeit dieser Einzelzellen zu untersuchen.

Dabei wurden zwei Varianten durchgeführt.

Im Experiment A wurde den Zellen nach initialer Adhäsion an die Petrischale "EFLA 85942" zugesetzt.

Im Experiment B wurde den Zellen unmittelbar vor dem Adhäsionsprozess "EFLA 85942" zugesetzt.

In diesen beiden Experimenten konnte eine statistisch signifikante Erniedrigung der Koloniebildungsfähigkeit festgestellt werden, die aber im Experiment B, im Vergleich zum Experiment A, in drastisch stärkerem Ausmass auftrat.

Das Ergebnis dieser beiden Experimente, zusammen mit der weiter oben beschriebenen Bildung von dreidimensionalen Zellaggregaten, lässt den Schluss zu, dass einerseits die Adhäsion mit nicht histo-kompatiblen Strukturen reduziert wird und dass andererseits die Adhäsion zwischen einzelnen, zum gleichen Gewebetyp gehörenden Zellen oder Zellverbänden gefördert wird.

Diese Verhaltensmerkmale beweisen, dass nebst der weiter oben genannten antioxidativen Wirkung der Polyphenole noch andere Wirkungsmechanismen und/oder weitere aktive Verbindungen eine wichtige Rolle spielen.

Die Behandlung des menschlichen oder tierischen Körpers kann darin bestehen, dass man die entsprechende Zubereitung einnimmt oder innerlich oder äusserlich anwendet.

Die Behandlung wird wenigstens so lange durchgeführt, bis die entsprechende Symptomatik verschwunden ist.

Die nachfolgenden Beispiele illustrieren die vorliegende Erfindung.

### Beispiel 1

Herstellung einer Zubereitung in der Form einer Hartgelatine-Kapsel.

Zur Herstellung von 1000 Kapseln der Grösse 1 wurden 100 g "EFLA 85942" mit 25 g mikrokristalliner Cellulose, 4 g Magnesiumstearat und 1 g gefällter Kieselsäure homogen vermischt.

Dieses Gemisch wurde in einer Menge von jeweils 150 mg in Hartgelatine-Kapseln abgefüllt.

### Beispiel 2

Herstellung einer Zubereitung in der Form einer Trinkampulle.

Zur Herstellung von 1000 Trinkampullen à 10 ml wurden 100 g "EFLA 85942" mit 15 g Kaliumsorbat, 15 g Natriumbenzoat, 500 g Fructose und 10 g Natriumchlorid homogen vermischt.

Dieses Gemisch wurde mit einer Lösung aus 95 Gewichtsteilen Wasser und 5 Gewichtsteilen Glycerol auf ein Gesamtvolumen von 10 Litern aufgefüllt, vermischt und steril filtriert.

Das erhaltene Filtrat wurde aseptisch in die Trinkampullen abgefüllt.

### Beispiel 3

Herstellung einer Zubereitung in der Form eines Liposomengels.

### Phase A

- 1,0 Gewichtsteile Rhodigel^{R} 200,
- 8,0 Gewichtsteile 1,2-Propylenglykol
wurden bei Raumtemperatur vermischt und während 1 Stunde quellen gelassen.

### Phase B

- 6,5 Gewichtsteile Phospholipon^{R} 80,
- 10,0 Gewichtsteile Polyethylenglykol 400,
- 2,0 Gewichtsteile Glycerol
wurden miteinander bei einer Temperatur von 60°C bis 70°C vermischt und homogenisiert.

Das erhaltene homogene Gemisch wurde unter Rühren auf eine Temperatur von 38°C bis 40° C abgekühlt.

### Phase C

Ein Gemisch aus
- 71,7 Gewichtsteile destilliertes Wasser,
- 1,2 Gewichtsteile "EFLA 85942"
wurde steril filtriert und dann tropfenweise in die Phase B eingearbeitet. Nach beendeter Hinzugabe wurde das erhaltene Gemisch auf Raumtemperatur abgekühlt, portionenweise in die Phase A eingearbeitet und homogenisiert.

Die erhaltene Zubereitung kann in Plastikspritzflaschen à 10 ml abgefüllt werden.

Die Zubereitung wurde unter aseptischen Bedingungen hergestellt.

### Anwendungsbeispiel 1

Eine 30-jährige weibliche gesunde Versuchsperson mit Kopfhaarproblemen in der Form von lichten Arealen und teilweise ergrauten Stellen nahm über eine Zeitspanne von 8 Wochen täglich 3 Kapseln gemäss Beispiel 1 ein.

Innerhalb dieser Zeitspanne veränderte sich der Zustand der Kopfbehaarung drastisch.

In den ergrauten Haarzonen sprossen normal gefärbte Haare, und die lichten Areale verschwanden.

### Anwendungsbeispiel 2

Eine 36-jährige männliche gesunde Versuchsperson mit Kopfhaarproblemen in der Form von lichten Arealen und teilweise ergrauten Stellen nahm über eine Zeitspanne von 8 Wochen täglich 6 Kapseln gemäss Beispiel 1 ein.

Innerhalb dieser Zeitspanne veränderte sich der Zustand der Kopfbehaarung drastisch.

In den ergrauten Haarzonen sprossen normal gefärbte Haare, und die lichten Areale verschwanden.

## Patentansprüche

1. Verwendung eines Teil- oder Vollextraktes aus nicht fermentierter Camellia sinensis L. zur Herstellung eines Medikamentes, eines Medizinproduktes, eines kosmetischen Präparates oder eines Nahrungsergänzungsproduktes, wobei diese Zubereitungen
- die Bildung von Nekrosen und/oder Atrophien in menschlichen oder tierischen Geweben und/oder das vorzeitige Absterben von vaskularisierten und nicht-vaskularisierten Zellen und Zellverbänden im menschlichen oder tierischen Körper verhindern oder zumindest wesentlich reduzieren, und
- sowohl die Adhäsion zwischen einzelnen, zum gleichen Gewebetyp gehörenden Zellen oder Zellverbänden fördern,
- als auch die Adhäsion zwischen einzelnen, zu verschiedenen nicht histo-kompatiblen Gewebetypen gehörenden Zellen oder Zellverbänden verhindern oder zumindest wesentlich reduzieren.

2. Verwendung nach Anspruch 1, dadurch gekennzeichnet, dass das Medikament oder das Medizinprodukt in einer pharmazeutisch akzeptablen Darreichungsform vorliegt, insbesondere in
- festen Darreichungsformen zur oralen Applikation, insbesondere in der Form einer Tablette, einer Filmtablette, eines Dragees, eines Pellets, einer Hartgelatine-Kapsel, einer Weichgelatine-Kapsel,
- flüssigen Darreichungsformen zur oralen, parenteralen, rektalen, vaginalen und topischen Applikation, insbesondere in der Form einer Tropflösung, eines Sprays, einer Injektionslösung, eines Sirups,
- halbfesten Darreichungsformen zur topischen, oralen, rektalen und vaginalen Applikation, insbesondere in der Form einer Crème, eines Gels, einer Salbe, einer Paste, eines Suppositoriums.

3. Verwendung nach Anspruch 1, dadurch gekennzeichnet, dass das kosmetische Präparat oder das Nahrungsergänzungsprodukt in der Form einer Lösung, eines Sprays, einer Crème, eines Gels, einer Salbe, einer Paste, eines Dragees, einer Kapsel, einer Ampulle oder eines Shampoos vorliegt.

4. Verwendung nach einem der Ansprüche 1 bis 3, dadurch gekennzeichnet, dass der Teil- oder Vollextrakt in Nano-Kapseln oder in Liposomen eingearbeitet ist.

5. Verwendung nach einem der Ansprüche 1 bis 4, dadurch gekennzeichnet, dass die genannten Zubereitungen noch wenigstens einen Zusatz- und/oder Hilfsstoff enthalten, insbesondere ausgewählt aus der Gruppe, bestehend aus Emulgatoren, Stabilisatoren, Antioxidantien, Farbstoffen, Aromen, Sprengmitteln, Lösungsmitteln, Gleitmitteln und Tensiden.

6. Verwendung nach einem der Ansprüche 1 bis 5, dadurch gekennzeichnet, dass der Teil- oder Vollextrakt in einer Menge von 0,1 Gew.-% bis 95 Gew.-%, bezogen auf das Gesamtgewicht der Zubereitung, enthalten ist.

7. Verwendung nach einem der Ansprüche 1 bis 6, dadurch gekennzeichnet, dass der Teil- oder Vollextrakt nebst nativen Polyphenolen und Purinalkaloiden noch wenigstens eine weitere native Verbindung, ausgewählt aus der Gruppe, bestehend aus Glutaminsäureethylamid, Glutaminsäure, einschliesslich deren physiologische Salze, und Glutamin, enthält.

8. Verwendung nach einem der Ansprüche 1 bis 7, dadurch gekennzeichnet, dass die genannten Zubereitungen zur Verhinderung, Behandlung oder Nachbehandlung von
- kachektischen Zuständen, bedingt durch Dünndarmzottenatrophien,
- Malabsorptionen, bedingt durch Dünndarmzottenatrophien,
- Polyneuropathien, bedingt durch nicht-entzündliche Axonschädigungen,
- Parodontosen, bedingt durch Gingiva-Zellwachstums- und Zell-Zell-Adhäsionsstörungen,
- Haarausfall, bedingt durch Haarwurzelatrophien,
- hypertrophen Hautveränderungen, beispielsweise Psoriasis vulgaris, Neurodermitis, bedingt durch Zell-Zell-Adhäsionsstörungen und Differenzierungsdefizite,
- Hyperplasien, bedingt durch pathologische Zellvermehrung, beispielsweise Hypersplenismus, bedingt durch Lymphogranulomatose, foveoläre Hyperplasie der Magenschleimhaut,
- Leukozyten-Reifungsstörungen, beispielsweise Leukämien der Untergliederungen
a.) akute lymphatische Leukämie
b.) chronische lymphatische Leukämie
c.) akute myeloische Leukämie, insbesondere Peroxydase-Typ,
d.) chronische myeloische Leukämie,
- exsudativen Formen der Lungentuberkulose,
- Lungenzirrhosen, beispielsweise chronische interstitielle Fibrose mit Parenchymschwund,
- Leberzirrhosen, beispielsweise als Folge von chronischer Hepatitis,
- Lungenemphysemen, oder
- Erythematodes visceralis,
dienen.

9. Verwendung nach einem der Ansprüche 1 bis 7, dadurch gekennzeichnet, dass die genannten Zubereitungen zur Behandlung menschlicher oder tierischer Zell- und/oder Gewebekulturen sowie Organen ausserhalb des menschlichen oder tierischen Körpers dienen, insbesondere Kultivierung und Vermehrung und/oder dreidimensionaler Aufbau von Knorpelzellen, Gingiva-Zellen, Haarwurzelzellen, Hautzellen und Hautgeweben, Netzhautzellen und Netzhautgeweben, Herzmuskelzellen und Herzmuskelgeweben, Leberzellen und Lebergeweben.
